# NEUE EUROPÄISCHE PATENTSCHRIFT

(11) **EP 1 148 867 B2**
(45) Veröffentlichungstag und Bekanntmachung des Hinweises auf die Entscheidung über den Einspruch: **03.10.2007**
(45) Hinweis auf die Patenterteilung: 07.05.2003
(21) Anmeldenummer: 00904906.5
(22) Anmeldetag: 13.01.2000
(51) Int. Cl.: A61K 8/41, A61K 8/42, A61K 8/37, A61Q 5/12

(54) **HAARPFLEGEMITTEL**
HAIR CARE PRODUCT
PRODUIT DE SOINS CAPILLAIRES

(30) Priorität: 04.02.1999 DE 19904359
(43) Veröffentlichungstag der Anmeldung: 31.10.2001
(73) Patentinhaber: Wella Aktiengesellschaft, 64274 Darmstadt (DE)
(72) Erfinder: PASQUIER, Gilbert, CH-1724 Praroman (CH); IRRGANG, Bernhard, CH-1713 St. Antoni (CH); MAGER, Herbert, CH-1723 Marly (CH)
(74) Vertreter: Fiesser, Gerold Michael
(86) Internationale Anmeldenummer: PCT/EP2000/000199
(87) Internationale Veröffentlichungsnummer: WO 2000/045787

(56) Entgegenhaltungen:
- EP-A- 0 166 232
- EP-A- 0 511 652
- EP-A- 0 566 049
- EP-A- 0 786 250
- WO-A-95/22313
- DE-A- 19 738 303
- DE-C- 19 727 903
- GB-A- 2 196 980
- US-A- 5 665 267
- GESSLEIN B W ET AL: "BEHENYL AND ISOSTEARYL QUATERNARIES. \FOR HAIR AND SKIN CARE APPLICATIONS" SOAP COSMETICS CHEMICAL SPECIALTIES,US,MAC.NAIR-DORLAND CO. NEW YORK, Bd. 66, Nr. 5, 1. Mai 1990 (1990-05-01), Seite 36,38,41,44,1 XP000133875 ISSN: 0091-1372
- Römpp Chemie lexikon, 9. Aufl., S. 350, 351, 628, 629, 1680-1687, 3730, 3731, 4168-4173
- Ullmann's Encyclopedia of Ind. Chem., 5. Aufl., Vol. A12, 1994, S. 576-581, 594, 595, 794, 795

## Beschreibung

Die vorliegende Erfindung betrifft ein Haarpflegemittel, mit einem Gehalt an einer Kombination eines quaternisierten Amins, insbesondere eines quaternisierten Amidoamins, und einem Ester aus einer aliphatischen Carbonsäure und einem Alkohol.

An kosmetischen Haarpflegemitteln werden hinsichtlich ihrer fachlichen Eigenschaften und der Umweltverträglichkeit ihrer Wirkstoffe immer höhere Anforderungen gestellt. Hierbei kommt es ganz entscheidend darauf an, daß das Mittel optimale Pflegeeigenschaften entfaltet, ohne daß unerwünschte Wirkungen beim Anwender und Belastungen der Umwelt auftreten.
Auch die Glanzwirkung bei Haaren soll erhöht werden. Außerdem muß ein solches Mittel noch den Anforderungen an Stabilität genügen und leicht zu handhaben sein.

Die bekannten kosmetischen Haarflegemittel, insbesondere die Sprüh- und Schaumkuren, enthalten als kationischen Pflegestoff üblicherweise das Kationtensid Distearyldimethylammoniumchlorid (DSDMAC, INCI-Bezeichnung: Quaternium-18).
DSDMAC wurde in der Vergangenheit in der Haarpflege breit eingesetzt und findet heute noch vor allem in Treatments, die besondere Pflegewirkungen aufweisen sollen (gute Pflege, geringe Belastung), vor allem in Verbindung mit polymeren Silikonverbindungen Verwendung. Der Nachteil dieser Substanz besteht vor allem in der schlechten biologischen Abbaubarket Außerdem besitzen sie eine hohe aquatische Toxizität und belasten die Umwelt stark. Wird DSDMAC durch andere übliche quaternäre Verbindungen wie Monoalklyquats (Cetyltrimethylammoniumchlorid) oder sog. Esterquats ersetzt, werden jedoch die anwendungstechnischen Eigenschaften des DSDMAC nicht erreicht.

Andererseits kann auf kationische Tenside in Haarpflegemitteln nicht verzichtet werden. Hierbei kann das Problem auftreten, daß im Falle flüssiger kosmetischer Mittel, welche insbesondere versprüh- oder verschäumbar sein sollen, einerseits eine geringe Viskosität und andererseits eine stabile Emulsion erreicht werden müssen. Die Verwendung von beispielsweise Distearyldimethylammoniumchlorid oder analogen quaternären Ammoniumverbindungen erfüllen zwar diese Bedingungen, jedoch kommt aufgrund der genannten Nachteile die Verwendung solcher Verbindungen in kosmetischen Mitteln nicht mehr in Betracht.

Aufgabe der vorliegenden Erfindung war es, Haarpflegemittel bereitszustellen, die die vorgenannten Nachteile nicht aufweisen, insbesondere frei sind von DSDMAC und/oder strukturverwandter Verbindungen, aber dennoch hervorragende haarpflegende Eigenschaften besitzen.

Die Aufgabe wurde erfindungsgemäß durch Bereitstellung eines Haarpflegemittels gemäß Anspruch 1 gelöst, welches dadurch gekennzeichnet ist, daß es eine Kombination enthält aus einem quaternisierten Amin der allgemeinen Formel I in einer Menge zwischen 0.01 und 10,0 Gewichtsprozent, 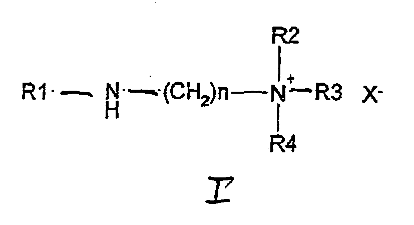 worin
R1 ein Acylrest oder ein Alkylrest mit 8 bis 24 Kohlenstoffatomen, welche verzweigt oder unverzweigt, gesättigt oder ungesättigt sein können, und wobei der Acylrest und/oder der Alkylrest mindestens eine OH-Gruppe enthalten können,
R2, R3 und R4 ein Alkylrest mit 1 bis 4 Kohlenstoffatomen, welcher gleich oder verschieden, gesättigt oder ungesättigt sein kann X⁻ ein Anion, n eine ganze Zahl zwischen 1 und 10, bedeuten,
und mindestens einem Ester einer aliphatischen, linearen oder verzweigten Carbonsäure mit einem primären oder sekundären, verzweigten oder unverzweigten Alkohol und mindestens einem Silikon und/oder Aminosilikon, einschließlich deren Derivate.

Bevorzugt wird ein Haarpflegemittel, worin das quaternisierte Amin gemäß allgemeiner Formel I ein quaternisiertes Amidoamin ist, worin R1 ein verzweigter oder unverzweigter, gesättigter oder ungesättigter Acylrest mit 8 bis 24 Kohlenstoffatomen, welcher mindestens eine OH-Gruppe enthalten kann, bedeutet.

Das erfindungsgemäße kosmetische Haarpflegemittel ist versprüh- oder verschäumbar und kann für alle bekannten kosmetischen Verwendungen, die üblicherweise die Haare betreffen, eingesetzt werden. Beispielsweise in Form einer Sprühkur, einer Schaumkur, einer Spülung oder eines Treatments.
Das erfindungsgemäße Haarpflegemittel kann nach Applikation auf das trockene, feuchte oder nasse Haar entweder im Haar verbleiben oder es kann nach einer geeigneten Einwirkzeit wieder ausgespült werden. Die Einwirkzeiten hängen von der Art des Haares ab. Als allgemeine Richtlinie kann aber von Einwirkzeiten zwischen 0,5 und 30 Minuten, insbesondere zwischen 0,5 und 10 Minuten, vorzugsweise zwischen 1,0 und 5,0 Minuten ausgegangen werden.

Überaschenderweise wurde gefunden, daß eine Kombination aus einem quaternisierten Amindoamin gemäß der allgemeinen Formel I und einem Ester einer aliphatischen, linearen oder verzweigten Carbonsäure mit einem primären oder sekundären, verzweigten oder unverzweigten Alkohol in hervorragender Weise geeignet ist, kosmetische Haarpflegmittel, vorzugweise flüssige Haarpflegemittel, mit hervorragenden haarpflegenden Wirkungen bereitzustellen. Überraschend war auch, daß die erfindungsgemäßen Haarpflegemittel gegenüber herkömmlichen Haarpflegemitteln überlegene Eigenschaften aufweisen und auch DSDMAC enthaltenden Haarpflegemitteln überlegen sind.

Bevorzugt werden für die erfindungsgemäße Kombination solche quaternisierten Amine, in denen R1 in der allgemeinen Formel I ein Fettsäurerest aus Ölen und Wachsen, insbesondere aus natürlichen Ölen und Wachsen, ist. Als Beispiele hierfür kommen Lanolin, Bienen- oder Candellilawachse in Betracht.

Bevorzugt sind auch solche quaternisierten Amine, in denen R2, R3 und/oder R4 in Formel I ein Rest gemäß der allgemeinen Formel CH₂CH₂OR5 bedeuten, worin R5 die Bedeutung von Alkylresten mit 1 bis 4 Kohlenstoffatomen, Hydroxyethyl oder H haben kann.
Die bevorzugte Größe von n in der allgemeinen Formel I ist eine ganze Zahl zwischen 2 und 5.

Weiterhin bevorzugt sind quaternisierte Amine der allgemeinen Formel I, in denen das Anion X⁻ ein Halogenidion oder eine Verbindung der allgemeinen Formel RSO₃⁻ ist, worin R die Bedeutung von gesättigtem oder ungesättigtem Alkylresten mit 1 bis 4 Kohlenstoffatomen hat.

Der Alkylrest mit 1 bis 4 Kohlenstoffatomen von R2, R3 und R4 und/oder der Alkylrest mit 1 bis 4 Kohlenstoffatomen von RSO₃⁻ in der allgemeinen Formel I können mindestens eine Hydroxylgruppe enthalten.

Als erfindungsgemäß zu verwendende quaternisierte Amidoamine, kommen beispielsweise in Betracht: REWOQUAT RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quaternium-33), REWOQUAT UTM 50 (Witco Surfactants GmbH, Undecyleneamidopropyltrimonium Methosulfate).

Die quaternisierten Amine gemäß der allgemeinen Formel I können einzeln oder in beliebigen Kombinationen miteinander verwendet werden, wobei Mengen zwischen 0,01 und 10,0 Gewichtsprozent, insbesondere zwischen 0,01 und 5,0 Gew.%, bezogen auf das fertige Produkt, eingesetzt werden.

Überraschenderweise konnte festgestellt werden, daß der Zusatz eines Esters aus einer aliphatischen, linearen oder verzweigten Carbonsäure mit einem primären oder sekundären, verzweigten oder unverzweigten (linearen) Alkohol zu einem quaternisiertem Amin gemäß der allgemeinen Formel I die besonders konditionierenden Eigenschaften des erfindungsgemäßen Haarpflegemittels im positiven Sinne starkt beeinflußt.
Es wurde nämlich unerwarteterweise gefunden, daß eine Kombination eines erfindungsgemäßen quaternisierten Amins mit mindestens einem erfindungsgemäßen Carbonsäureester ein Haarpflegemittel bereitstellen läßt, welches ausgezeichnete pflegenden und haarkonditionierenden Eigenschaften entfaltet. Insbesondere werden hierdurch der Griff, die Kämmbarkeit von sowohl feuchten als auch trockenem Haar, sowie die Geschmeidigkeit gegenüber herkömmlichen Haarpflegemitteln wesentlich verbessert. Darüberhinaus zeichnet sich ein solches Haarpflegemittel durch sehr gute Umweltverträglichkeit aus.
Der Kombination aus einem erfindungsgemäßen quaternisierten Amin und mindestens einem der genannten Ester kommt daher erfindungsgemäß hohe Bedeutung zu.

Die vorteilhaften Eigenschaften des Esters der besagten Carbonsäuren werden insbesondere mit Estern einer aliphatischen, linearen oder einer verzweigten Carbonsäure mit 8 bis 18 Kohlenstoffatomen erzielt. Demzufolge ist ein Haarpflegemittel gemäß der vorliegenden Erfindung bevorzugt, worin der Ester einer aliphatischen, linearen oder einer verzweigten Carbonsäure 8 bis 18 Kohlenstoffatome aufweist.

Der mit der Carbonsäure veresterte primäre oder sekundäre, verzweigte oder unverzweigte Alkohol weist vorteilhafterweise 1 bis 6 Kohlenstoffatome auf.

Der für die Esterbildung primäre oder sekundäre, verzweigte oder unverzweigte Alkohol ist bevorzugt ein Monoalkohol.
Ein ganz besonders bevorzugter Carbonsäure Ester ist Isopropylmyristat.

Der Ester kann in einer Menge von 0,01 bis 20,0 Gewichtsprozent, vorzugsweise in einer Menge von 0,01 bis 10,0 Gewichtsprozent, insbesondere in einer Menge von 0,01 bis 5,0 Gewichtsprozent, eingesetzt werden.

Erfindungsgemäß enthält das Haarpflegemittel ein oder mehrere Silikone und/oder Aminosilikone oder deren Derivate, einzeln oder als Gemisch.
Hiermit wird eine weitere Verbesserung der Pflegewirkung erreicht.

Geeignete Silikonderivate umfassen solche der allgemeinen Formel (II), 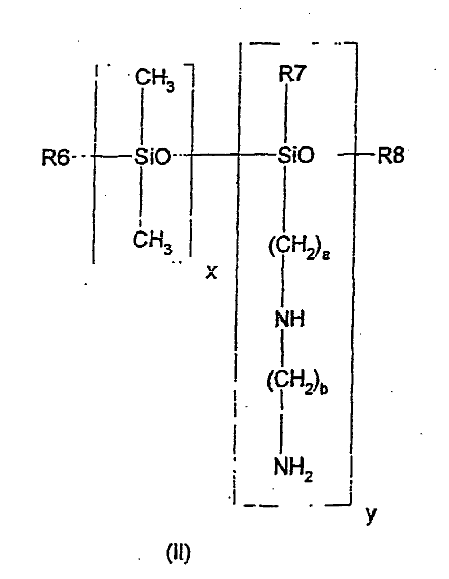 worin R6 Trimethylsilyl oder OH, R7 Methyl oder OH, R8 Trimethylsilyl oder H, a eine ganze Zahl zwischen 1 und 8, b eine ganze Zahl zwischen 1 und 5, x und y ein beliebiger ganzzahliger Wert, bedeuten.

Insbesondere sind aminofunktionelle Silikone gemäß allgemeiner Formel (II) geeignet, in der a eine ganze Zahl zwischen 2 und 4 und b eine ganze Zahl zwischen 1 und 3 bedeuten.

Diese aminofunktionellen Silikone haben ein Molekulargewicht zwischen 2000 und 100000 und sind beispielsweise von Dow Coming unter der Bezeichnung Silicone Emulsion No. 929, mit einem Gehalt an aminofunktionellen Silikon von ca. 35%, zu erhalten (R6, R7 = OH, R8 = H). Ebenso kann beispielsweise ein aminofunktionelles Silikon von Toshiba mit der Bezeichnung XF42-B1989 (Aktivsubstanz: 100 %, R6, R8 = Trimethylsilyl und R7 = Methyl) Verwendung finden. Ein solches Silikon ist erfindungsgemäß ganz besonders bevorzugt.

Ein weiteres bevorzugtes aminofunktionelles Silikon, das für das erfindungsgemäße Haarpflegemittel verwendet werden kann, ist ein Silikon gemäß Strukturformel (III), 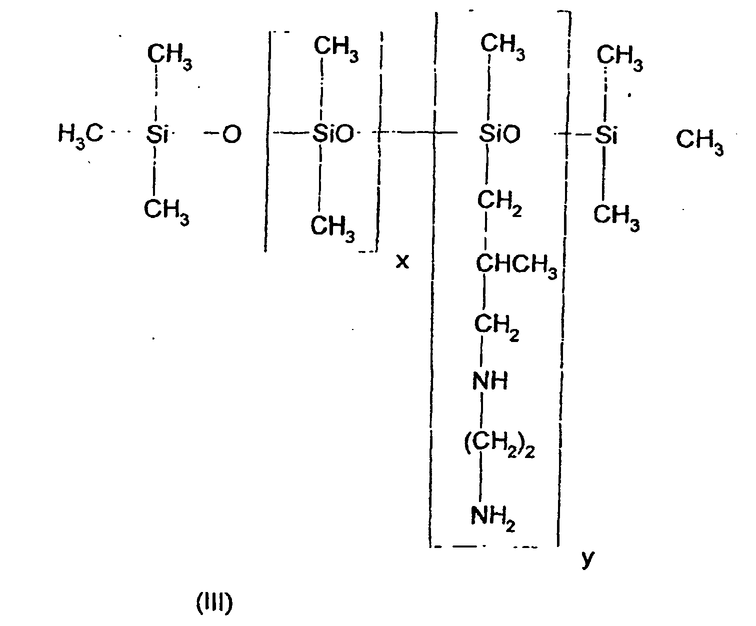 wenn das Verhältnis x zu y zwischen etwas 27.5 : 1 bis zu ungefähr 160 : 1 variieren kann. Das Molekulargewicht einer solchen Verbindung liegt dann zwischen 5000 und etwa 30000. Ein derartiges Produkt ist von Dow Coming unter der Bezeichnung Amodimethicone Q2-8075 im Handel, das ein Molekulargewicht von 8000 aufweist und ein Verhältnis von x zu y in Formel (III) von etwa 49:1 aufweist. Dieses Amodimethicone ist in dem genannten Produkt zu etwa 37% enthalten.

Desweiteren sind in vorteilhafter Weise Silikone der allgemeinen Formel (IV) als Zusatz für das erfindungsgemäße Haarpflegemittel geeignet, 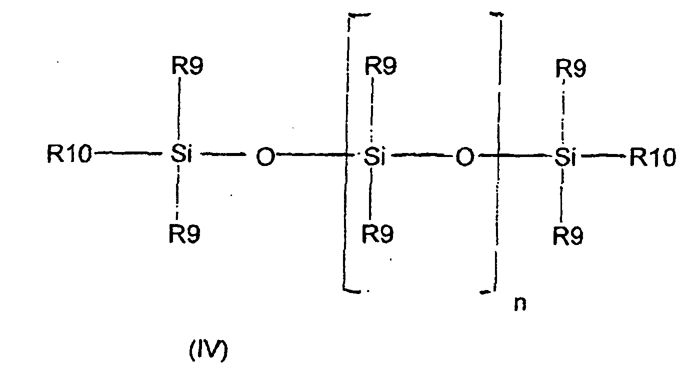 worin R9 Methyl oder OH, R10 Methyl oder Phenyl und n eine ganze Zahl bedeuten.

Die in der allgemeinen Formel (IV) genannte Verbindung soll vorzugsweise eine Viskosität von 1000 bis 1000000 cSt aufweisen. Besonders geeignet sind Emulsionen von Silikonen, die z.B. von der Fa. Toshiba Silicones unter der Bezeichnung XS65-B3803 vertrieben werden, wo ein Silikon gemäß allgemeiner Formel (IV) mit einer Viskosität von ca. 100000 cSt eingesetzt wird (Aktivgehalt: ca 30%). Als weitere Beispiele sind zu nennen: BY 22-050A, BY22-060, BY22-062 bzw. BY22-047 von Toray Silicones.

Die Silicone und/oder Aminosilikone können als Einzelstoffe oder in Kombination miteinander in einer Gesamtkonzentration von 0,01 bis 10,0 Gewichtsprozent, vorzugsweise in einer Konzentration von 0,01 bis 8,0 Gewichtsprozent, bezogen auf das Silikon, eingesetzt werden.

Dem erfindungsgemäßen Haarpflegemittel kann desweiteren mindestens ein Konsistenzgeber hinzugefügt werden.
Als geeignete Konsistenzgeber kommen in Betracht: Fettalkohole, insbesondere solche mit 8 bis 18 Kohlenstoffatomen, vorzugsweise Cetyl- oder Cetearylalkohol, sowie Reaktionsprodukte dieser Fettalkohole mit 1 bis 10 Mol Ethylenoxid oder Propylenoxid oder ihren Mischungen, Cellulose bzw. deren Derivate wie zum Beispiel Alkylhydroxyalkylcellulosen, beispielsweise Tylose C-Typen von Clariant, Methylhydroxyethylcellulose, beispielsweise Tylose MH-Typen von Clariant, Hydroxyethylcellulose, beispielsweise Tylose H-Typen von Clariant oder Natrosol 250-Typen von National Starch, Hydroxypropylmethylcellulose, beispielsweise Methocel-Typen von Dow Chemical, Silica bzw. sogenannte "Hydrated Silica", die beispielsweise von Degussa unter den Handelsnamen Aerosil bzw. Sipemat vertrieben werden, oder neutrale Polymere, wie beispielsweise langkettiges Polyvinylpyrrolidon (PVP).

Im allgemeinen können die Konsistenzgeber in einer Konzentration von 0,1 bis 15,0 Gewichtsprozent, bevorzugt in einer Menge von 0,1 bis 10,0 Gewichtsprozent, bezogen auf die Gesamtmenge des fertigen Haarpflegemittels, eingesetzt werden.

Es hat sich herausgestellt, daß dem Haarpflegemittel weitere vorteilhafte haarpflegende Wirkung verliehen wird, wenn zusätzlich mindestens ein kationischen Tensid zugegeben wird, ausgenommen Distearyldimethylammoniumchlorid und Analoga hiervon.
Erfindungsgemäß kann für das Haarpflegemittel ein kationisches Tensid der allgemeinen Formel (V) verwendet werden, 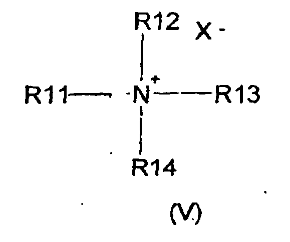 worin R11 und R12 gleich oder verschieden sein können und entweder einen verzweigten oder unverzweigten Alkylrest mit 1 bis 4 Kohlenstoffatomen bedeuten, welcher mindestens eine Hydroxylgruppen enthalten kann, oder einen gesättigten oder ungesättigten, verzweigten oder unverzweigten Alkylrest mit 8 bis 22 Kohlenstoffatomen bedeuten, welcher mindestens eine Hydroxylgruppe enthalten kann, oder eine Verbindung der allgemeinen Formel (Va) bedeuten, 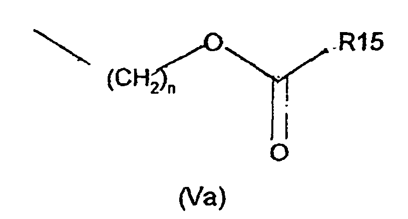 in der R15 ein gesättigter oder ungesättigter, verzweigter oder unverzweigter Alkylrest mit 8 bis 22 Kohlenstoffatomen ist, welcher mindestens eine Hydroxylgruppe enthalten kann, und n eine ganze Zahl zwischen 1 bis 4 bedeutet, R13 und R14 einen verzweigten oder unverzweigten Alkylrest mit 1 bis 4 Kohlenstoffatomen darstellen, welcher mindestens eine Hydroxylgruppe enthalten kann; und X⁻ ein beliebiges Anion bedeutet, vorzugsweise Chlorid oder Methosulfat. Ausgenommen sind Verbindungen mit R11 und R12 mit der Bedeutung von Alkylresten mit 8 bis 22 Kohlenstoffatomen und mit R13 und R14 mit der Bedeutung von Alkylresten mit 1 bis 4 Kohlenstoffatomen (DSDMAC-Typen).

Bevorzugte Verbindungen sind zum Beispiel Cetyltrimethylammoniumchlorid (Arquad 16 von Akzo Nobel), oder auch sogenannte Esterquats, deren Herstellung in den europäischen Patentanmeldungen EP 0 284 036 A2, EP 0 669 391 A2 und EP 0 550 361 A1 beschrieben ist, und deren Verwendung zur Pflege von keratinischen und anderen Fasern aus der internationalen Patentanmeldung WO 96/03970 A1, und den europäischen Patentanmeldungen EP 0 636 356 A1, EP 0 689 532 A1 und EP 0 614 349 A1 bekannt ist. Die Verbindungen sind beispielsweise unter den Handelsnamen REWOQUAT WE 18, REWOQUAT WE 38 DPG (Witco Surfactants GmbH), Stepantex GS 90 (Stepan), Armocare VGH-70 (Akzo Nobel), oder Dehyquart L-80 (Henkel) erhältlich.
Die kationischen Tenside können in Mengen von 0,01 bis 5,0 Gewichtsprozent, vorzugsweise in Mengen von 0,1 bis 3,0 Prozent, bezogen auf die Gesamtmenge des erfindungsgemäßen Haarpflegemittels, verwendet werden.
Weiterhin können für das erfindungsgemäße Haarpflegemittel sogenannte Betainester in üblichen Mengen eingesetzt werden, wie sie in der deutschen Patentanmeldung DE 35 27 974A1 beschrieben sind.

Selbstverständlich kann das erfindungsgemäße Haarpflegemittel alle für Haarkosmetika üblichen und bekannten Hilfs-, Wirk- und Zusatzstoffe enthalten.

Grundsätzlich ist dem Fachmann bekannt, welche Hilfs- bzw. Trägerstoffe, Wirk- und Zusatzstoffe -, in der Haar- und Hautkosmetik verwendet werden, so daß die näheren Ausführungen nur beispielhaften Charakter haben und nur zur weiteren Veranschaulichung der vorliegenden Erfindung dienen sollen.
Im übrigen kann hierzu auf die vorliegende Literatur verwiesen werden, die den allgemeinen Aufbau von Haarpflegemitteln beschreiben, beispielsweise SCHRADER, K., Grundlagen und Rezepturen der Kosmetika, 2. Auflage, 1989, Seiten 728 - 737 oder DOMSCH, A., Die kosmetischen Präparate, Verlag für chemische Industrie (H. Ziolkowsky, Ed.), 4. Auflage, Band 2 Seiten 212 - 230, 1992 oder JOHNSON, D. H. (Hrsg.), Hair and Hair Care, New York, 1997, Seiten 65 - 104.

Als übliche Hilfs-, Wirk- und Zusatzstoffe, die in dem erfindungsgemäßen Haarpflegemittel verwendet werden können, können beispielsweise genannt werden: kationische oder wasserlösliche, nicht-ionische Polymere, pflanzliche und mineralische Fette und Öle, Parfümöle, in der Haarkosmetik übliche Farbstoffe, weitere haarkonditionierende Mittel wie synthetische und natürliche Phospholipide, quaternäre Derivate der Stärke oder Cellulose, Lösungsvermittler wie beispielsweise kurzkettige primäre und sekundäre Alkohole (zum Beispiel Ethanol, 1- oder 2-Propanol), Proteine oder Proteinderivate wie beispielsweise Proteinhydrolysate (zum Beispiel Kollagen-, Keratin-, Seidenprotein- oder Weizenproteinhydrolysate), Aminosäuren (zum Beispiel Histidin, Glycin, Alanin, Serin, Threonin, Arginin, Cystein und deren Derivate, beispielsweise der Fettsäurekondensationsprodukte), Pflanzenextrakte, Vitamine oder Provitamine (zum Beispiel Biotin, Vitamin C, D-Panthenol der Derivate davon), Allantoin, Chitosan, viskositätsregulierende Stoffe wie beispielsweise Fettsäurealkanolamide, alkoxylierte Ester von Polyolen (zum Beispiel Glycerin, Sorbitol, Fructose oder Glucose, Antischuppenmittel, anorganische oder organische Säuren (zum Beispiel Essigsäure, Milchsäure, Citronensäure, Glykolsäure, Äpfelsäure, Phosphorsäure), Sonnenschutzmittel bzw. UV-Absorber, Konservierungsmittel, Antioxidantien (zum Beispiel Tocopherole oder Ester davon).

Aus der Gruppe der kationischen oder wasserlöslichen, nicht-ionischen Polymere eignen sich vor allem Poly-dialkyl-diallylammoniumverbindungen, Polyvinylpydrrolidon, Copolymere aus Vinylpyrrolidon und Dialkylaminoalkylmethacrylat oder ein quatemisiertes Copolymere aus Vinylpyrrolidon und Dialkylaminoalkylmethacrylat, beispielsweise Dimethyldiallylammoniumchlorid-Polymer (Merquat 100 (Calgon), Polyvinylpyrrolidon (Luviskol K 80 (BASF)), Copolymere aus Vinylpyrrolidon und Dimethylaminoethylmethacrylat (Copolymer 845, Copolymer 937, Copolymer 958 (ISP) mit Diethylsulfat quatemisierte Copolymere aus Vinylpyrrolidon und Dimethylaminoethylmethacrylat (Gafquat 734, Gafquat 755 N oder Luviquat PQ 11).

Die Zusatz-, Hilfs- und Trägerstoffe können in den für den Fachmann bekannten üblichen Mengen verwendet werden, insbesondere in Mengen von 0,1 bis 10,0 Gewichtsprozent.

Der pH-Wert des erfindungsgemäßen Haarpflegemittels liegt vorzugsweise im neutralen bis sauren Bereich, bevorzugt zwischen pH 2,0 und 6,0. Der pH-Wert kann gegebenenfalls mit den bekannten Hilfsmitteln eingestellt werden. Hierfür eignen sich insbesondere die in kosmetischen Produkten üblichen anorganischen oder organischen Säuren, beispielsweise Phosphorsäure, Zitronensäure, Milchsäure, Äpfelsäure, Glykolsäure oder Essigsäure.

Das erfindungsgemäße Haarpflegemittel kann in den für flüssige Haarpflegemittel bekannten Applikationsformen vorliegen, insbesondere als Spülung, Sprüh- und Schaumkur oder Treatment. Das erfindungsgemäße Haarpflegemittel kann nach Aufbringen auf das Haar dort verbleiben oder ausgespült werden. Bevorzugt wird ein Haarpflegemittel, welches nach der Applikation ausgespült wird.

Das erfindungsgemäße Haarpflegemittel kann - je nach eingestellter Viskosität - vorteilhafterweise mit Hilfe üblicher Pumpen und Dosierhilfen auf das trockene oder feuchte Haar aufgebracht bzw. aufgesprüht oder verschäumt werden, beispielsweise als Aerosol. Geeignete Versprüh- oder Verschäumvorrichtung sind dem Fachmann bekannt, welche mit einem Treibmittel oder mit Hilfe mechanisch verursachter Druckerzeugung betrieben werden können. Die Auftragung erfolgt vorzugsweise im Anschluß an eine Haarwäsche.

Wenn das erfindungsgemäße Haarpflegemittel mit Hilfe eines Treibmittels appliziert, insbesondere versprüht oder verschäumt, werden soll, so kann es zusammen mit üblichen Treibmitteln in einen geeigneten Druckbehälter, insbesondere in eine Aerosoldose, in üblichen Verhältnissen oder Mengen abgefüllt werden. Als Beispiel kann hierfür ein Verhältnis von Haarpflegemittel zu Treibmittel wie 92 : 8 angegeben werden. Der Innendruck ist abhängig von dem verwendeten Behälter. Als Richtlinie kann bei einer Temperatur von 20° C von einem Druck im Bereich von 1,5 bis 8,0 bar ausgegangen werden.

Als Treibmittel sind beispielsweise niedere Alkane, wie zum Beispiel n-Butan, i-Butan und Propan, Dimethylether, sowie gasförmig vorliegende Treibmittel, wie beispielsweise N₂, N₂O oder CO₂, sowie Gemische der vorstehend genannten Treibmittel, geeignet.

Unter mechanischen Versprüh- oder Verschäumvorrichtungen sind solche Vorrichtungen zu verstehen, welche das Versprühen oder Verschäumen ohne Verwendung eines Treibmittels ermöglichen. Als geeignete mechanische Versprühoder Verschäumvorrichtungen kann beispielsweise eine Pumpe oder ein mit einem Sprühventil versehener elastisch verformbarer Behälter, in den das erfindungsgemäße Haarpflegemittel abgefüllt wird, verwendet werden. Im Falle, daß die Abfüllung unter Druck erfolgt, kann das erfindungsgemäße Haarpflegemittel nach Öffnen des Versprüh- oder Verschäumventils kontinuierlich oder dosiert abgegeben werden. Im anderen Fall kann der zum Austritt des erfindungsgemäßen Mittels erforderliche Druck durch reversibles mechanisches Verformen des Behälters erzeugt werden.

Aufgrund der beschriebenen hervorragenden Eigenschaften der erfindungsgemäßen Kombination aus einem quaternisierten Aminoamin gemäß der vorliegenden Beschreibung mit mindestens einem Ester einer aliphatischen, linearen oder verzweigten Carbonsäure und einem Alkohol gemäß vorliegender Beschreibung eignet sich diese Kombination in vorteilhafterweise zur Herstellung von allgemein kosmetischen Präparationen, welche pflegende und/oder reinigende und/oder konditionierende Eigenschaften, insbesondere an Hautanhangsgebilden, entfalten sollen. Beispielsweise kann die erfindungsgemäße Kombination zur Herstellung von Mascarapräparationen, Augenbrauenpräparationen, Haarstylingmittel, Haarkonditioniermittel, Haarverformungsmitteln, Haarfärbemitteln, Haarvor- und Haarnachbehandlungs-mitteln, Tonika bzw. Haarwässer oder Deodorantien verwendet werden.
Auch kosmetische Präparationen enthaltend eine Kombination aus einem quaternisierten Amin mit mindestens einem Ester einer aliphatischen, linearen oder verzweigten Carbonsäure und einem Alkohol und mindestens einem Silikon und/oder Aminosilikon, einschließlich deren Derivate gemäß vorliegender Beschreibung, werden daher von der vorliegenden Erfindung mitumfaßt.

Das erfindungsgemäße Haarpflegemittel wird durch die nachfolgenden Beispiele A, B und D bis G näher erläutert und herkömmlichen Haarpflegemitteln (Vergleichsbeispiele C und H bis L) gegenübergestellt. Die in den Tabellen angegebenen Zahlen verstehen sich als Gewichtsprozent, bezogen auf die Gesamtmenge, wenn nicht anders erläutert.

Bei der Durchführung der vergleichenden nachfolgenden Versuche wurde das Haupthaar der Probanden mit einem handelsüblichen milden Shampoo ohne konditionierende oder pflegende Wirkung wie üblich gewaschen. Dann wurde das Testprodukt auf das handtuchfeuchte Haar gegeben. Nach einer Einwirkzeit von ca. 3 Minuten wurde das Produkt wieder ausgespült. Die Beurteilung im Frisiersalon erfolgte über Halbseitentests; beim Endverbrauchertest wurde das Produkt auf dem ganzen Kopf angewendet.

**Tabelle I:**

| Beispiele: | | | | | | | |
|---|---|---|---|---|---|---|---|
| | A | B | C* | D | E | F | G |
| Cetearylalkohol | 8 | 8 | 8 | 7.5 | 8 | 8 | |
| Hydroxypropylmethylcellulose | | | | | | | 4 |
| Paraffin | | | | | 2 | 2 | |
| Lanolin | 2 | 2 | 2 | 2.6 | | | 1 |
| Lanoquat DES-50 | 2 | | | 1 | 1 | 1 | 2 |
| REWOQUAT RTM 50 | | 2 | | | | | |
| Adogen S18V | | | 1.5 | | | | |
| Isopropylmyristat | 2 | 2 | 3 | 2 | 2 | 2 | 2 |
| Dimethicon-Emulston¹ | 1 | 3 | | | 0.8 | | |
| TMS-Amodimethicon² | | | 1.3 | 1.6 | 0.8 | | 1.5 |
| TMS-Amodimethicon-Emulsion³ | | | | | | 1 | |
| Amodimethicon⁴ | | | | | | 0.2 | |
| THA-Chlorid | 2.6 | 1.3 | 1.3 | 1.3 | 1.3 | 2 | |
| TEA-Esterquat | | | | | | | 1.5 |
| Polyquat-11 | | | 0.2 | | | 0.3 | |
| Parfüm | 0.1 | 0.1 | 0.1 | 0.1 | 0.1 | 0.1 | 0.1 |
| Zitronensäure | ad pH4 | ad pH4 | ad pH4 | ad pH4 | ad pH4 | ad pH4 | ad pH4 |
| Wasser | ad 100 | ad 100 | ad 100 | ad 100 | ad 100 | ad 100 | ad 100 |

| | | | | | | | |
|---|---|---|---|---|---|---|---|
| * Vergleichsbeispiel ¹: Die Emulsion enthielt 30 % hochmolekulares Dimethicon, ca. 40 % niedrigviskoses Methylpolysiloxan, Emulgatoren und Wasser ²: 100%ig, Toshiba XF 49-B1989 ³: Dow Corning 939 ⁴: Dow Corning Q2-8075 | | | | | | | |

**Tabelle II:**

| Vergleichsbeispiele | | | | | |
|---|---|---|---|---|---|
| | H | I | J | K | L |
| Cetearylalkohol | 8 | 8 | 8 | 8 | |
| Hydroxypropylmethylcellulose | | | | | 4 |
| Paraffin | | | 2 | | |
| Lanolin | 2 | 2 | | 2 | 1 |
| Lanoquat DES-50 | | | 1 | | 2 |
| REWOQUAT RTM 50 | | | | | |
| Adogen S18V | | | | 1.5 | |
| Isopropylmyristat | | 2 | | | |
| Dimethicon-Emulsion¹ | | 1 | 0.8 | | |
| TMS-Amodimethicon² | | | 0.8 | 1.3 | |
| TMS-Amodimethicon-Emulsion³ | | | | | |
| Amodimethicon⁴ | 1 | | | | |
| DSDMAC | 1.5 | | | | |
| THA-Chlorid | 2.6 | 4 | 1.3 | 1.3 | 1.3 |
| Polyquat-11 | | | | 0.2 | |
| Parfüm | 0.1 | 0.1 | 0.1 | 0.1 | 0.1 |
| Zitronensäure | ad pH4 | ad pH4 | ad pH4 | ad pH4 | ad pH4 |
| Wasser | ad 100 | ad 100 | ad 100 | ad 100 | ad 100 |

**Tabelle III:**

| Resultate der Halbseitenversuche | | | | | |
|---|---|---|---|---|---|
| | Griff feucht | Kämmbarkeit feucht | Griff trocken | Kämmbarkeit trocken | Geschmeidigkeit |
| A gegen H | gleich | gleich | A besser | A besser | A besser |
| A gegen I | A besser | A besser | A besser | A besser | A besser |
| D gegen H | D besser (glatter) | D besser | D besser (weicher, (glatter) | D besser | D besser |
| E gegen J | E besser (glatter) | gleich | E besser (weicher, glatter) | E besser | E besser |
| C gegen K | C besser | gleich | C besser (weicher) | C besser | C besser |
| G gegen L | G besser | G besser | G besser (weicher) | G besser | G besser |

Vor allem bei den Kriterien für das trockene Haar weisen die erfindungsgemäßen Beispiele Vorteile auf. Selbst gegenüber einer Rezeptur, die DSDMAC enthält, liegen für das erfindungsgemäße Haarpflegemittel eindeutige Vorteile vor (Vergleichsversuche A gegen H und D gegen H).

Bei einem Endverbrauchertest, bei dem die Probanden zwei Produkte zur Beurteilung bekamen, schnitt das erfindungsgemäße Beispiel D am besten ab.

## Patentansprüche

1. Haarpflegemittel enthaltend eine Kombination aus einem quaternisierten Amin der allgemeinen Formel (I) in einer Menge zwischen 0,01 und 10,0 Gewichtsprozent 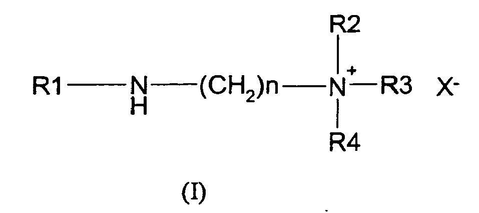 worin
R1 ein Acylrest oder Alkylrest mit 8 bis 24 Kohlenstoffatomen, welche verzweigt oder unverzweigt, gesättigt oder ungesättigt sein können, wobei der Acylrest und/oder der Alkylrest mindestens eine OH-Gruppe enthalten können,
R2, R3 und R4 ein Alkylrest mit 1 bis 4 Kohlenstoffatomen, welcher gleich oder verschieden, gesättigt oder ungesättigt sein kann,
X⁻ ein Anion,
n eine ganze Zahl zwischen 1 und 10,
bedeuten,
und mindestens einem Ester einer aliphatischen, linearen oder verzweigten Carbonsäure mit einem primären oder sekundären, verzweigten oder
unverzweigten Alkohol und mindestens einem Silikon und/oder Aminosilikon, einschließlich deren Derivate.

2. Haarpflegemittel nach Anspruch 1, **dadurch gekennzeichnet, daß** R2, R3 und/oder R4 ein Rest gemäß der allgemeinen Formel CH₂CH₂OR5 ist, worin R5 die Bedeutung von Alkylresten mit 1 bis 4 Kohlenstoffatomen, Hydroxyethyl oder H haben kann.

3. Haarpflegemittel nach Ansprüchen 1 und 2, **dadurch gekennzeichnet, daß** R1 ein Fettsäurerest aus Ölen und Wachsen ist.

4. Haarpflegemittel nach Ansprüchen 1 bis 3, **dadurch gekennzeichnet, daß** das Anion X⁻ ein Halogenidion oder eine Verbindung der allgemeinen Formel RSO₃⁻ ist, worin R die Bedeutung von gesättigtem oder ungesättigtem Alkylresten mit 1 bis 4 Kohlenstoffatomen hat.

5. Haarpflegemittel nach Ansprüchen 1 bis 4, **dadurch gekennzeichnet, daß** der Alkylrest mit 1 bis 4 Kohlenstoffatomen von R2, R3 und R4 und/oder der Alkylrest mit 1 bis 4 Kohlenstoffatomen von RSO₃⁻ mindestens eine Hydroxylgruppe enthält.

6. Haarpflegemittel nach Ansprüchen 1 bis 5, **dadurch gekennzeichnet, daß** der Ester einer aliphatischen, linearen oder einer verzweigten Carbonsäure 8 bis 18 Kohlenstoffatome aufweist.

7. Haarpflegemittel nach Ansprüchen 1 bis 6, **dadurch gekennzeichnet, daß** der mit der Carbonsäure veresterte primäre oder sekundäre, verzweigte oder unverzweigte Alkohol 1 bis 6 Kohlenstoffatome aufweist.

8. Haarpflegemittel nach Ansprüchen 1 bis 7, **dadurch gekennzeichnet, daß** der primäre oder sekundäre, verzweigte oder unverzweigte Alkohol ein Monoalkohol ist.

9. Haarpflegemittel nach Ansprüchen 1 bis 8, **dadurch gekennzeichnet, daß** das Silikon ausgewählt aus den Verbindungen der allgemeinen Formeln (II), (III) und (IV), 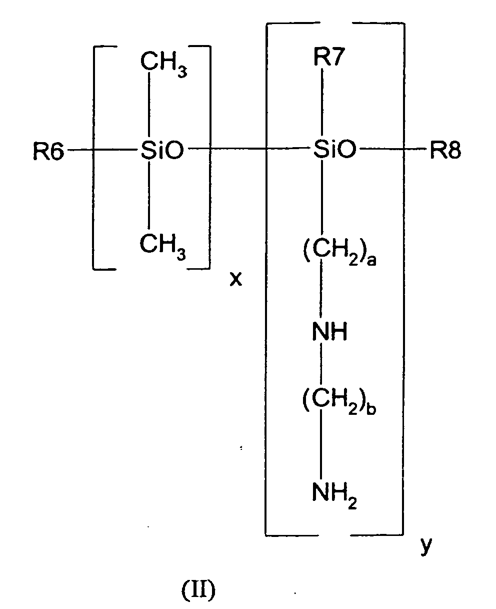 worin
R6 Trimethylsilyl oder OH,
R7 Methyl oder OH,
R8 Trimethylsilyl oder H,
a eine ganze Zahl zwischen 1 und 8
b eine ganze Zahl zwischen 1 und 5,
x und y ein beliebiger ganzzahliger Wert,
bedeuten, 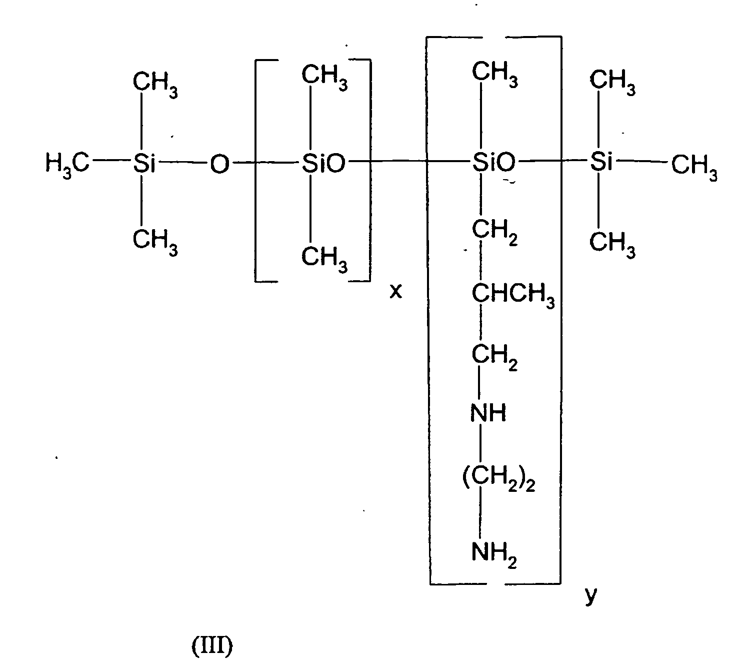 worin
x und y ein beliebiger ganzzahliger Wert bedeuten, 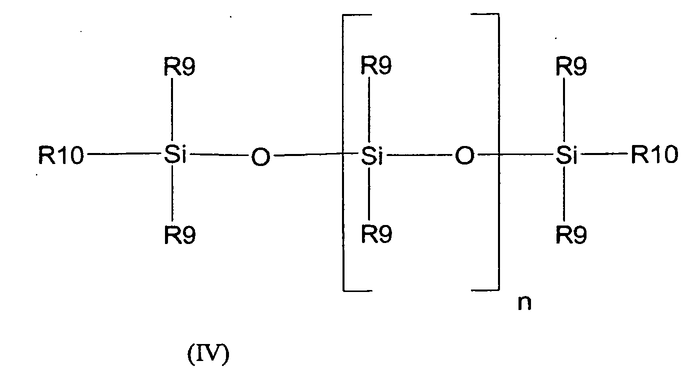 worin
R9 Methyl oder OH,
R10 Methyl oder Phenyl,
n = ein beliebiger ganzzahliger Wert,
bedeuten.

10. Haarpflegemittel nach Ansprüchen 1 bis 9, **dadurch gekennzeichnet, daß** es zusätzlich mindestens einen Konsistenzgeber enthält.

11. Haarpflegemittel nach Ansprüchen 1 bis 10, **dadurch gekennzeichnet, daß** es zusätzlich mindestens ein kationisches Tensid enthält, ausgenommen Distearyldimethylammoniumchlorid und Analoga hiervon.

12. Haarpflegemittel nach Anspruch 11, **dadurch gekennzeichnet, daß** das kationische Tensid eine Verbindung gemäß der allgemeinen Formel (V) ist, 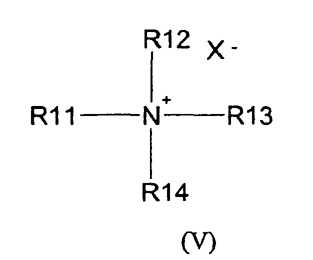 worin
R11 und R12 gleich oder verschieden sein können und entweder ein verzweigter oder unverzweigter Alkylrest mit 1 bis 4 Kohlenstoffatomen ist, welcher mindestens eine Hydroxylgruppen enthalten kann, oder ein gesättigter oder ungesättigter, verzweigter oder unverzweigter Alkylrest mit 8 bis 22 Kohlenstoffatomen ist, welcher mindestens eine Hydroxylgruppe enthalten kann,
R13 und R14 einen verzweigten oder unverzweigten Alkylrest mit 1 bis 4 Kohlenstoffatomen,
X⁻ ein Anion,
oder eine Verbindung gemäß der allgemeinen Formel (Va), 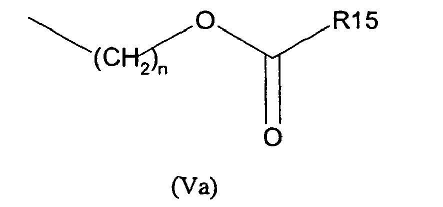 in der
R15 ein gesättigter oder ungesättigter, verzweigter oder unverzweigter Alkylrest mit 8 bis 22 Kohlenstoffatomen ist, welcher mindestens eine Hydroxylgruppe enthalten kann,
und n eine ganze Zahl zwischen 1 und 4 ist,
bedeuten.

13. Kosmetische Präparation enthaltend eine Kombination aus einem quaternisierten Amin der allgemeinen Formel (I) in einer Menge zwischen 0,01 und 10,0 Gewichtsprozent 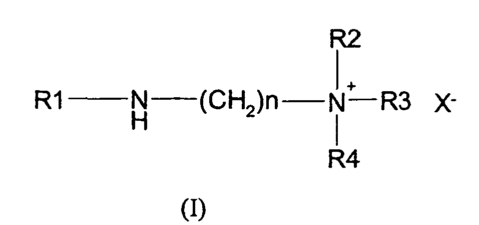 worin
R1 ein Acylrest oder Alkylrest mit 8 bis 24 Kohlenstoffatomen, welche verzweigt oder unverzweigt, gesättigt oder ungesättigt sein können, wobei der Acylrest und/oder der Alkylrest mindestens eine OH-Gruppe enthalten können,
R2, R3 und R4 ein Alkylrest mit 1 bis 4 Kohlenstoffatomen, welcher gleich oder verschieden, gesättigt oder ungesättigt sein kann,
X⁻ ein Anion,
n eine ganze Zahl zwischen 1 und 10,
bedeuten,
und mindestens einem Ester einer aliphatischen, linearen oder verzweigten Carbonsäure mit einem primären oder sekundären, verzweigten oder
unverzweigten Alkohol und mindestens einem Silikon und/oder Aminosilikon, einschließlich deren Derivate.

## Claims

1. Haircare composition comprising a combination of a quatemized amine of the general formula (I) in an amount between 0.01 and 10.0% by weight. 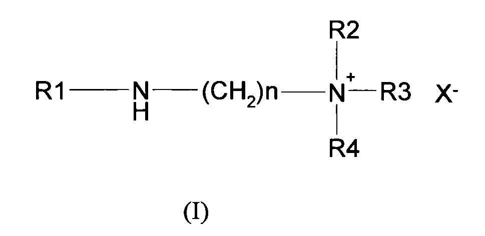 in which
R1 is an acyl radical or alkyl radical having 8 to 24 carbon atoms, which may be branched or unbranched, saturated or unsaturated, where the acyl radical and/or the alkyl radiacal may contain at least one OH group, R2, R3 and R4 are an alkyl radical having 1 to 4 carbon atoms which may be identical or different, saturated or unsaturated,
X⁻ is an anion,
N is an integer between 1 and 10,
and at least one ester of an aliphatic, linear or branched carboxylic acid with a primary or secondary, branched or unbranched alcohol and at least one silicone and/or aminosilicone, including derivatives thereof.

2. Haircare composition according to Claim 1, **characterized in that** R2, R3 and/or R4 is a radical according to the general formula CH₂CH₂OR5, in which R⁵ can have the meaning of alkyl radicals having 1 to 4 carbon atoms, hydroxyethyl or H.

3. Haircare composition according to Claims 1 and 2, **characterized in that** R1 is a fatty acid radical from oils and waxes.

4. Haircare composition according to Claims 1 to 3, **characterized in that** the anion X⁻ is a halide ion or a compound of the general formula RSO₃⁻, in which R has the meaning of saturated or unsaturated alkyl radicals having 1 to 4 carbon atoms.

5. Haircare composition according to Claims 1 to 4, **characterized in that** the alkyl radical having 1 to 4 carbon atoms of R2, R3 and R4 and/or the alkyl radical having 1 to 4 carbon atoms of RSO₃- contains at least one hydroxyl group.

6. Haircare composition according to Claims 1 to 5, **characterized in that** the ester of an aliphatic, linear or a branched carboxylic acid has 8 to 18 carbon atoms.

7. Haircare composition according to Claims 1 to 6, **characterized in that** the primary or secondary, branched or unbranched alcohol esterified with the carboxylic acid has 1 to 6 carbon atoms.

8. Haircare composition according to Claims 1 to 7, **characterized in that** the primary or secondary, branched or unbranched alcohol is a monoalcohol.

9. Haircare composition according to Claims 1 to 8, **characterized in that** the silicone is chosen from the compounds of the general formulae (II), (III) and (IV), 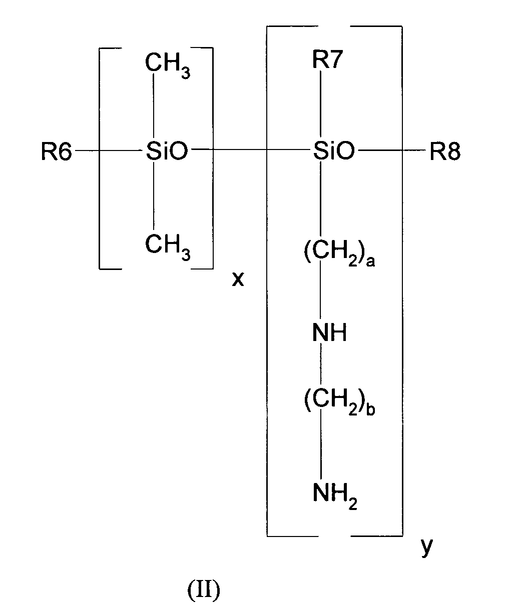 in which
R6 is trimethylsilyl or OH,
R7 is methyl or OH,
R8 is trimethylsilyl or H,
a is an integer between 1 and 8,
b is an integer between 1 and 5,
x and y are an arbitrary integer, 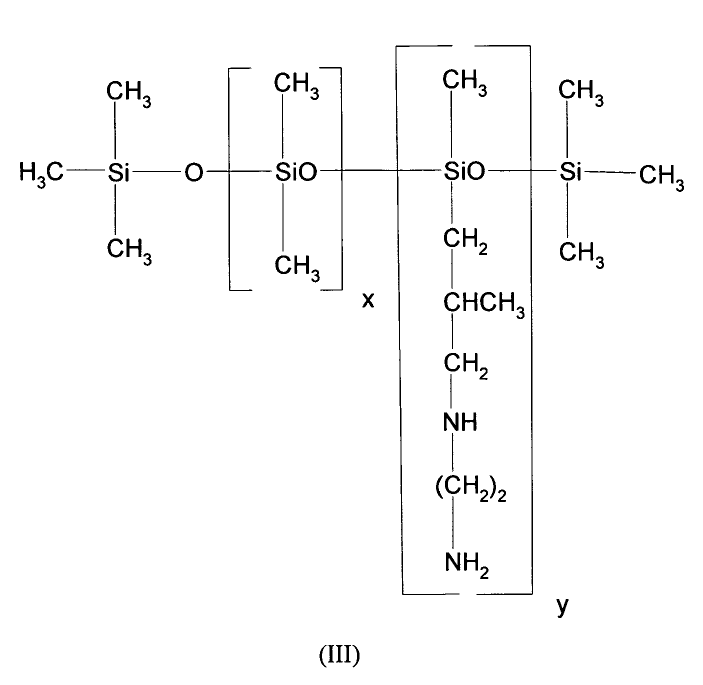 in which
x and y are an arbitrary integer, 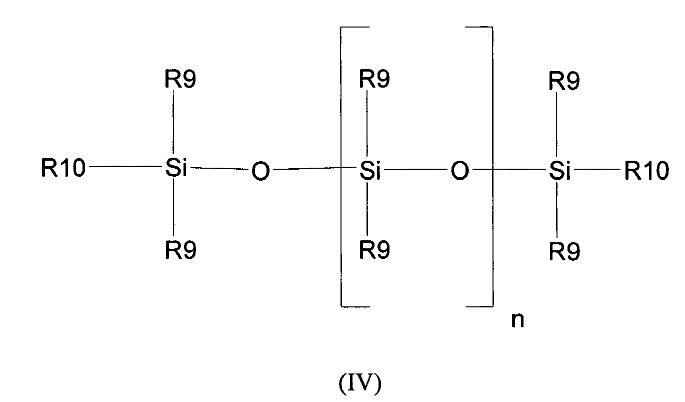 in which
R9 is methyl or OH,
R10 is methyl or phenyl,
n = an arbitrary integer.

10. Haircare composition according to Claims 1 to 9, **characterized in that** it additionally comprises at least one bodying agent.

11. Haircare composition according to Claims 1 to 10, **characterized in that** it additionally comprises at least one cationic surfactant, with the exception of distearyldimethylammonium chloride and analogues thereof.

12. Haircare composition according to Claim 11, **characterized in that** the cationic surfactant is a compound according to the general formula (V), 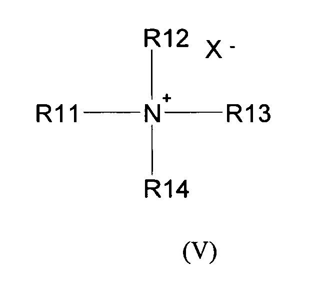 in which
R11 and R12 may be identical or different and are either a branched or unbranched alkyl radical having 1 to 4 carbon atoms which can contain at least one hydroxyl group, or are a saturated or unsaturated, branched or unbranched alkyl radical having 8 to 22 carbon atoms which can contain at least one hydroxyl group, R13 and R14 are a branched or unbranched alkyl radical having 1 to 4 carbon atoms,
X⁻ is an anion,
or a compound according to the general formula (Va), 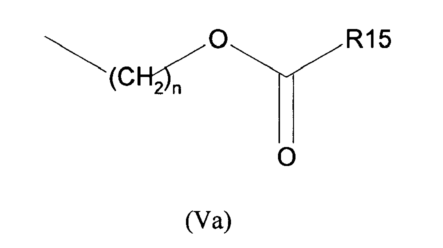 in which
R15 is a saturated or unsaturated, branched or unbranched alkyl radical having 8 to 22 carbon atoms which can contain at least one hydroxyl group,
and n is an integer between 1 and 4.

13. Cosmetic preparation comprising a combination of a quatemized amine of the general formula (I) in an amount between 0.01 and 10.0% by weight, 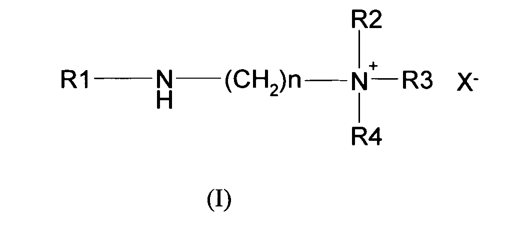 in which
R1 is an acyl radical or alkyl radical having 8 to 24 carbon atoms, which may be branched or unbranched, saturated or unsaturated, where the acyl radical and/or the alkyl radical may contain at least one OH group, R2, R3 and R4 are an alkyl radical having 1 to 4 carbon atoms which may be identical or different, saturated or unsaturated,
X⁻ is an anion,
n is an integer between 1 and 10,
at least one ester of an liphatic, linear or branched carboxylic acid with a primary or secondary, branched or unbranched alcohol and at least one silicone and/or aminosilicone, including derivatives thereof.

## Revendications

1. Composition de soin capillaire contenant une combinaison d'une amine quatemisée de fomule générale (I) dans une quantité comprise entre 0.01% et 10.0% de poids, 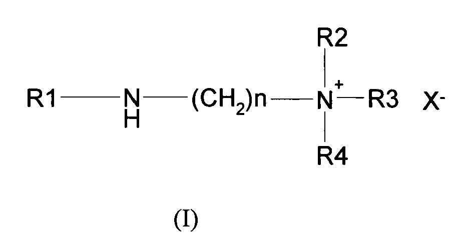 dans lesquelles représente
R1 un radical acyle ou un radical alkyl avec 8 à 24 atomes de carbone, qui peuvent être ramifiés ou non ramifiés, saturés ou insaturés, le radical acyle et/ou le radical alkyle pouvant contenir au moins un groupe OH, R2, R3 et R4 représentent un radical alkyle avec 1 à 4 atomes de carbone, qui peut être identique ou différent, saturé ou insaturé,
X⁻ représente un anion,
n représente un nombre entire compris entre 1 et 10,
et d'au moins un ester d'un acide carboxylique aliphatique, linéaire ou ramifié, avec un alcool primaire ou secondaire, ramifié ou non ramifié et au moins une silicone et/ou une aminosilicone, y compris leurs dérivés.

2. Composition de soin capillaire selon la revendication 1, characterisée en ce que R2, R3 et/ou R4 représentent un radical selon la formule générale CH₂CH₂OR5, dans laquelle R5 peut représenter de radicaux alkyle avec 1 à 4 atomes de carbone, un hydroxyéthyle ou H.

3. Composition de soin capillaire selon les revendications 1 et 2, characterisée en ce que R1 est un radical d'acide gras à base d'huiles et de cires.

4. Composition de soin capillaire selon les revendications 1 à 3, characterisée **en ce que** l'anion X⁻ est un ion halogénure ou un compose de la formule générale RSO₃⁻, dans laquelle R représente des radicaux alkyle saturés ou insaturés avec 1 à 4 atomes de carbone.

5. Composition de soin capillaire selon les revendications 1 à 4, characterisée en ce que le radical alkyle avec 1 à 4 atomes de carbone de R2, R3 et R4 et/ou le radical alkyle avec 1 à 4 atomes de carbone de RSO₃⁻ contient au moins un groupe hydroxyle.

6. Composition de soin capillaire selon les revendications 1 à 5, characterisée en ce que l'ester d'un acide carboxylique aliphatique, linéaire ou d'un acide carboxylique ramifié contient 8 à 18 atomes de carbone.

7. Composition de soin capillaire selon les revendications 1 à 6, characterisée en ce que l'alcool primaire ou secondaire esterifié avec l'acide carboxylique, ramifié ou non ramifié contient 1 à 6 atomes de carbone.

8. Composition de soin capillaire selon les revendications 1 à 7, characterisée en ce que l'alcool primaire ou secondaire, ramifié ou non ramifié, est un monoalcool.

9. Composition de soin capillaire selon les revendications 1 à 8, characterisée en ce que la silicone est sélectionnée parmi les composes des formules générale (II), (III) et (IV), 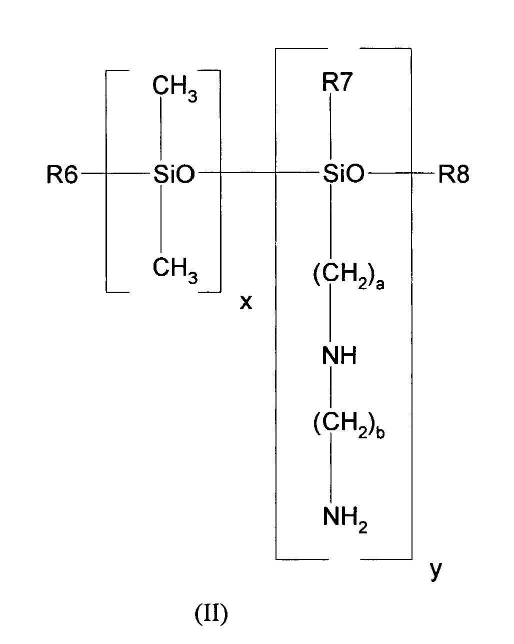 dans lesquelles
R6 représente du triméthylsilyle ou OH,
R7 du méthyle ou OH,
R8 du triméthylsilyle ou H,
a un nombre entier compris entre 1 et 8,
b un nombre entier compris entre 1 et 5,
x et y un nombre entier quelconque, 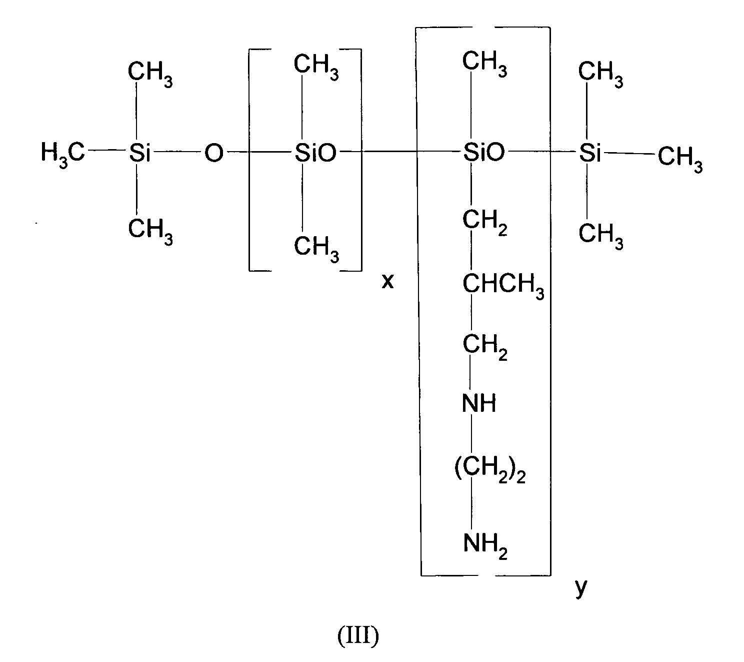 dans lesquelles x et y représentent une valeur entière quelconque, 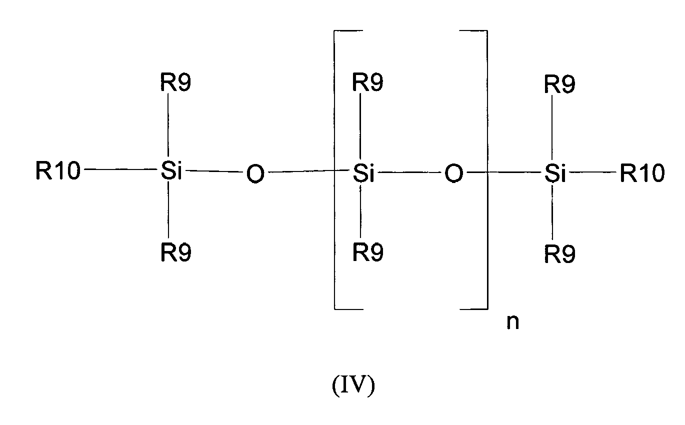 dans lesquelles R9 représente du méthyle ou OH,
R10 du méthyle ou du phényl,
n une valeur entière quelconque.

10. Composition de soin capillaire selon les revendications 1 à 9, characterisée en ce qu'il contient en supplément au moins un générateur de consistence.

11. Composition de soin capillaire selon les revendications 1 à 10, characterisée en ce qu'il contient en supplément au moins un tensioactif cationique, à l'exception du chlorure de distéaryldiméthylammonium et des produits similaires à celui-ci.

12. Composition de soin capillaire selon la revendication 11, characterisée en ce que le tensioactif cationique est un compose selon la formule générale (V), 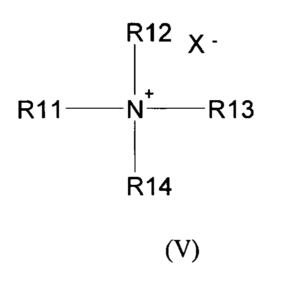 dans laquelle
R11 et R12 peuvent être identiques ou différents et représentent un radical alkyle ramifié ou non ramifié avec 1 à 4 atomes de carbone, lequel peut contenir au moins un groupe hydrxyle, ou bien représentent un radical alkyle saturé ou insaturé, ramifié ou non ramifié avec 8 à 22 atomes de carbone, lequel peut contenir au moins un groupe hydroxyle,
R13 et R14 peuvent contenir un radical alkyle ramifié ou non ramifié avec 1 à 4 atomes de carbone,
X⁻ représente un anion,
ou un compose selon la formule générale (Va), 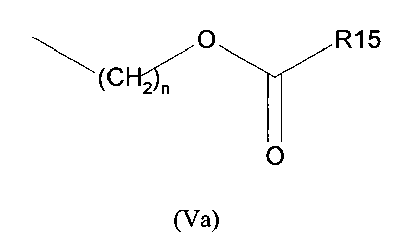 dans laquelle
R15 est un radical alkyle saturé ou insaturé, ramifié ou non ramifié avec
8 à 22 atomes de carbone, lequel peut contenir au moins un groupe hydroxyle,
et n représente un nombre entire compris entre 1 et 4.

13. Préparation cosmétique contenant une combinaison composée d'une amine quatemisée de formule générale (I) dans une quantité comprise entre 0,01 % et 10,0 % de poids, 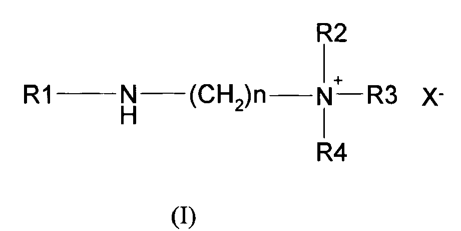 dans lesquelles
R1 représente un radical acyle ou alkyle avec 8 à 24 atomes de carbone, qui peuvent être ramifiés ou non ramifies, saturé ou insaturés, le radical acyle et/ou le radical alkyle pouvant contenir au moins un groupe OH,
R2, R3 et R4 représentent un radical alkyle avec 1 à 4 atomes de carbone, lequel peut être identique ou different, saturé ou insaturé,
X⁻ représente un anion,
n un nombre entire compris entre 1 et 10,
et d'au moins un ester d'un acide carboxylique aliphatique, linéaire ou ramifié avec un alcool primaire ou secondaire, ramifié ou non ramifié et au moins une silicone et/ou une aminosilicone, y compris leurs derives.
